# EUROPEAN PATENT APPLICATION

(11) **EP 2 219 126 A1**
(43) Date of publication of application: **18.08.2010**
(21) Application number: 10153148.1
(22) Date of filing: 10.02.2010
(51) Int. Cl.: G06F 19/00

(54) **Method and system for monitoring a personal intake**

(30) Priority: 12.02.2009 US 378487
(71) Applicant: Medixine Oy, 02600 Espoo (FI)
(72) Inventor: Jokinen, Tapio, 02100 Espoo (FI)
(74) Representative: Brax, Matti Juhani

(57) **Abstract**

An identifier comprising readable ID information related to the identifier is associated to a product or product type so that when selecting said product the corresponding identifier ID is read and transferred to a server as a query by a user related communication device, which may be e.g. a mobile station or cash machine. The server is configured to be in a data communication connection with a database comprising identifier ID and content information of each product or product type. The transferred identifier ID is then connected with content information of the product or product type with corresponding identifier ID at the database, after which said content information is transferred as a response to said query to said user related communication device.

## Description

### TECHNICAL FIELD OF THE INVENTION

The invention relates to a method and system for monitoring a personal intake, such as intake or consumption of nutrition.

### BACKGROUND OF THE INVENTION

Content of a consumed nutrition plays an important role e.g. for persons following a special diet, such as weight-watchers, or persons having some limitation or recommendations for consumed substances. Monitoring the consumption is often considered difficult, laborious or time-consuming.

Solutions for informing the user of nutrients contained in the nutrition are previously known. For example different computer programs and data terminal equipment are further known for monitoring the supply of nutrients of a person, for example, during dieting or weight watching. For example, the publication US 5,412,564 discloses a solution, in which the nutrition consumption of a consumer can be monitored and information concerning nutrition consumption can be recorded. The publication US 5,233,520 again discloses a solution for an interactive, computerised measuring apparatus of nutrition, which can be used for measuring the food, nutrients and other food components consumed by a person.

In addition the publication US 2003163354 discloses a solution for a portable nutritional monitoring device wherein nutritional data can be scanned, entered and stored directly from product packaging. Furthermore the publication US 2005025864 discloses a solution for monitoring or controlling and recording a nutritional intake of a subject comprising providing a plurality of different types of foods packaged to contain a predetermined and substantially uniform content of at least one nutritional component; and monitoring or controlling the number of food packages consumed during a predetermined time period.

However, there is still a clear need for an easy solution for monitoring accurate personal intake, as well as a need for arranging a flexible solution how to easily and fast gather and manage data needed for monitoring personal consumption, such as personal cumulative value or personal daily value.

### SUMMARY OF THE INVENTION

The present invention offers a solution e.g. for monitoring an accurate personal intake independently of a location and providing an easy method for controlling e.g. the limitations of calories during a diet. In addition the invention provides an easy method for changing e.g. content information of products to be consumed.

An arrangement for monitoring a personal intake of products according to the invention is provided, wherein
- the arrangement comprises identifiers each associated to a product, said identifier comprising readable identifier ID related to said identifier,
- the arrangement comprises a user related communication device having reading means for reading said identifier ID and sending means for transferring said read identifier ID to a server as a query, said server being in a data communication connect with a first database, where said first database comprises identifier ID and content information of each product or product type,
- the arrangement is configured to connect said transferred identifier ID with content information of the product or product type with corresponding identifier ID at the first database and to send said content information as a response to said query to said user related communication device.

A method for monitoring a personal intake of products according to the invention is provided, comprising
- associating an identifier to a product, said identifier comprising readable ID information related to said identifier,
- reading and transferring said identifier ID to a server as a query by a user related communication device, said server being in a communication connect with a first database, where said first database comprises identifier ID and content information of each product or product type,
- connecting said transferred identifier ID with content information of the product or product type with corresponding identifier ID at the first database and transferring said content information as a response to said query to said user related communication device.

An user related communication device for monitoring a personal intake of products according to the invention is provided , wherein the user related communication device is configured to
- read identifier ID, said identifier being associated to a product and comprising readable identifier ID related to said identifier,
- send said read identifier ID to a server as a query, said server being in a data communication connect with a first database comprising identifier ID and content information of each product or product type,
- receive content information as a response to said query, where said content information is provided by connecting said transferred identifier ID with content information of the product or product type with corresponding identifier ID at the first database.

A server for monitoring a personal intake of products according to the invention is configured to
- receive an identifier ID as a query sent by a user related communication device, where said ID relates to an identifier associated to one product,
- be in a data communication connect with a first database, where said first database comprises identifier ID and content information of each product or product type,
- connect said transferred identifier ID with content information of the product or product type with corresponding identifier ID at the first database and send said content information as a response to said query to said user related communication device.

A computer readable medium for monitoring a personal intake of products according to the invention is configured to
- interpret identifier ID read by a user related communication device, said identifier being associated to a product and comprising readable identifier ID related to said identifier,
- send said read identifier ID to a server as a query, said server being in a data communication connect with a first database comprising identifier ID and content information of each product or product type,
- receive content information as a response to said query, where said content information is provided by connecting said transferred identifier ID with content information of the product or product type with corresponding identifier ID at the first database,
when said computer readable medium is run on the user related communication device.

According to an embodiment of the invention identifiers comprising readable ID information related to the identifier are provided so that each identifier is associated to one product or product type. When selecting said product e.g. to be consumed the corresponding identifier ID is read and transferred to a server as a query by a user related communication device, which may be e.g. a mobile station or cash machine. The server is configured to be in a data communication connection with a first database, where said first database comprises identifier ID and content information of each product or product type. According to the embodiment said transferred identifier ID is connected with content information of the product or product type with corresponding identifier ID at the first database and said content information is transferred as a response to said query to said user related communication device. It is also possible in an embodiment that identifier IDs of all selected products are transferred at the same time to the server and/or content information relating to all products corresponding said plurality of identifier IDs is gathered at the server and transferred as cumulative data to said query to said user related communication device.

According to another embodiment also user ID is transferred to the server and a consumption database of the consumption of said user is kept by associating said user ID with content information of the product corresponding to the transferred identifier ID. This allows to transfer user related consumption information derived from the consumption database based on said transferred user ID e.g. to the user related communication device and/or to a second database to which the user has access. The derived consumption information may be e.g. personal cumulative value or personal daily/monthly value, but also cumulative data of plurality of products selected at a certain event.

According to an embodiment the consumption database may comprise additional information of the user, such as original weight, age, height, sex, current activity level and how much weight is desired to lose, accumulated radiation dose and/or toxic substances. Then e.g. an indication of the calories intake, such as calories still left for that day and/or an indication of radiation dose, and/or amounts of toxic substances can be calculated based on this additional information as well as nutritional content information of the product related to said transferred identifier ID. These data may be provided as personal data since user ID identifying each user is used. The indication can then be transferred to the user related communication device and/or to the second database. According to an embodiment the additional information can also at least partly be sent as a part of the query, or it may be stored in the consumption database beforehand.

The identifier associated to products may be e.g. an RFID tag, bar code or smart code (2D bar code) and according to an embodiment they comprise only readable ID information related to the identifier. On the contrary the identifier does not comprise any information about the product to which said identifier is associated. The product information is only on a database, such as on said first database. This has an advantage that if the content information about the product in question changes, e.g. radiation dose, amounts of toxic of other information, it has to be changed only at the database and the identifier associated to said product remains untouched.

According to an embodiment ID information can be transferred to the server application e.g. using an SMS, voice or other similar message or data connection.

### BRIEF DESCRIPTION OF THE DRAWINGS

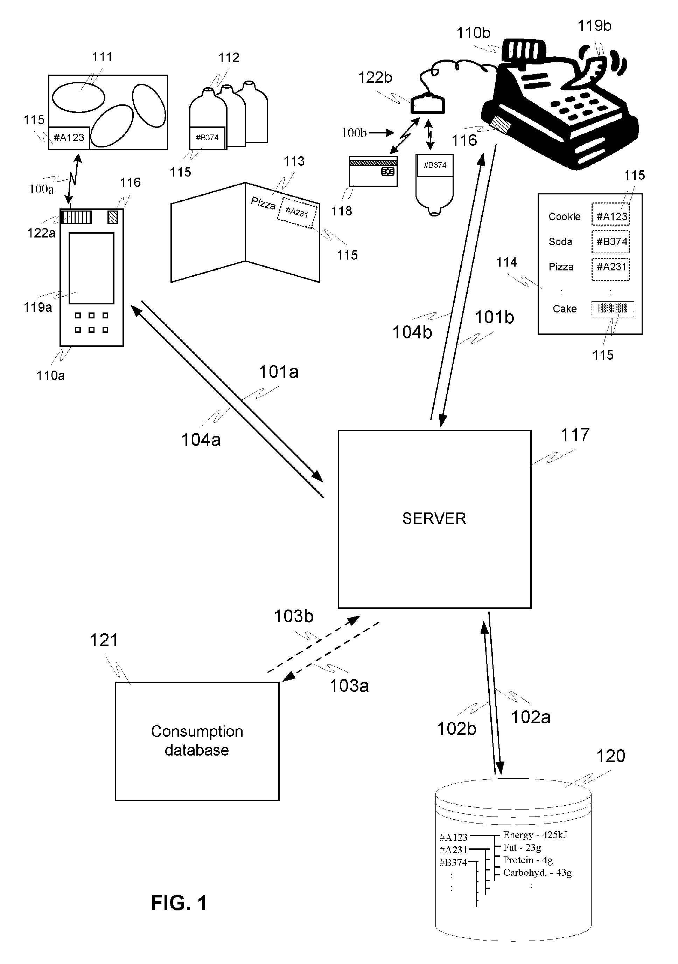

Next the invention will be described in greater detail with reference to exemplary embodiments in accordance with the accompanying figure 1, which illustrates an exemplary arrangement for monitoring a personal intake of products.

### DETAILED DESCRIPTION

Figure 1 illustrates an overall outlook of the invention, such as devices, communication connections, and method steps for data flow between the entities for enabling monitoring personal intake or consumption e.g. of substances, radiation doses and amount of toxic included in products. Each product or product type, such as cookies 111, soda bottles 112 or items in a menu 113, 114, is associated with identifier 115 comprising readable ID information (e.g. #A123, #B374, #A231) related to the identifier. The identifier may be a part of the product like printed smart/bar code on the soda bottle (#B374) or otherwise associated to the product like a RFID tag (#A123) attached to a plate on which the products (e.g. cookies) are offered.

According to an embodiment the identifier 115 is read 100a, 100b by a user related communication device, such as e.g. a personal mobile station 110a or non-personal cash machine 110b, wherein the user related communication device comprises e.g. RFID reader, bar code reader, and/or smart code reader 122a, 122b. Reading 100a may be done by the consumer by his mobile station 110a when he is selecting the product for example by reading the bar code attached into the product 112 or by reading the RFID tag integrated into the menu 115 in connection with the product to be ordered, like pizza. Reading 100b may also be done by the vendor using the cash machine 110b so that when the consumer has collected the products the identifiers 115 attached into the products 112 are read directly from the identifier on the product 112 or by reading the corresponding identifier from the menu or list 114 representing the product by the reading means 122b.

The user related communication device 110a, 110b is advantageously provided with suitable client application 116 interpreting the read identifier ID or all IDs together e.g. if plurality of the products is selected and sending 101 a, 101 b to a server 117 as a query. After receiving the identifier ID or all IDs at the same time the server makes a query 102a to a first database 120. The first database comprises identifier ID and content information of each product or product type. The server or database is then configured to connect the identifier ID received from the device 110a, 110b with content information of the product or product type with corresponding identifier ID at the first database. For example if the identifier ID was #A123, the corresponding content information is represented by the first database e.g. as: "Energy 425kJ, Fat, 23g, Protein 4g".

The content information of the product corresponding said received identifier ID is then transferred 102b to the server, which again sends 104a, 104b it as a response to said query to the user related communication device 110a, 110b. Again the client application (implemented e.g. by a suitable computer program) 116 at the user related communication device may interpret the content information received and e.g. display 119a it to the user or print it 119b. According to an embodiment content information relating to all products corresponding plurality of identifier IDs is gathered at the server or database and transferred as cumulative data to the user related communication device. Again gathering may also be done by the client application 116 at the user related communication device, which then represents gathered content information of the selected products as a cumulative data over the all selected products.

According to another embodiment also user ID may be sent to the server at step 101 a, 101b, whereupon a consumption database 121 of the consumption of said user corresponding said user ID may be kept and updated. The consumption database, which may be e.g. a personal health record (PHR), may be updated e.g. by associating said user ID with content information of the product corresponding to said transferred identifier ID at the server 117 so that after the step 102b the server may update the consumption database of the user in question by sending 103a the content information of the product in question to the consumption database 121, where the content information is associated with the user ID of the user in question. Updating may be completed automatically or after accepting by the user. The server 117 may also gather 103b consumption history data related to the user ID from the consumption database 121, such as e.g. personal cumulative value or personal daily value, and communicate it to the user related communication device 110a, 110b.

The consumption database 121 may further comprise (received in connection with the query and/or stored beforehand and/or gathered another way) additional information of the user, such as original weight, age, height, sex, current activity level and how much weight is desired to lose, accumulated radiation dose and/or toxic substances, for example. Based on the additional information as well as nutritional content information of the product related to the identifier ID the server 117 may be configured to calculate for example an indication of the calories intake, such as calories still left for that day and/or an indication of radiation dose, and/or amounts of toxic for a user based on his user ID received at step 101 a, 101b. The server 117 is then also configured to transfer 104a, 104b the indication at least to the user related communication device 110a, 110b. Again the application 116 at the user related communication device may interpret the indication information received and e.g. display 119a it to the user or print it 119b in an understandable form, such e.g. "maximum limit exceeded" or "10% still left of the day limit".

One exemplary embodiment may concern loosing weight, when the server 117 may be configured to calculate e.g. a personal daily limit so that the desired weight is still possible to be attained. The server may apply for example the following rules:
Basal energy expenditure (BEE):
   amount of energy required to maintain the body's normal metabolic activity, i.e. respiration, maintenance of body temperature etc.
   Male: BEE = 66.67 + 13.75W + 5H - 6.76A
   Female: BEE = 665.1 + 9.56W +1.85H -4.68A.
   H= height in cm , W= weight in kg, A= age in years.
   Maintain current weight (calories) = BEE x activity factor
   Maximum calories allowed that promote weight loss:
   (BEE x activity factor) - 500
whereupon the server may for example calculate the maximum calories allowed for each day and compare the already consumed calories and send indication about this information, which the application 116 on the user related communication device 110a, 110b interprets and displays in understandable form.

The user ID may be any kind of information by which each user can be individualized at least in connection to said query, such as ID information of the user related communication device, like phone number when the device is user's mobile station, or IP-number of the user related communication device when the device is e.g. a cash machine. Of course in the cash machine example the IP-number can be used for identifying the user only during the transaction and not longer, but often this is enough. Another possibility is that user ID is ID information input to the user related communication device, such as user name, password and/or social security number for example. Again the ID information may be read from the outer source, such as an ID card 118.

According to an embodiment the identifier of the product, as well as also user ID, can be read by the cash machine 110b, and send 101 b them to the server 117 as a query, whereupon the server 117 may based on the user ID find the user's mobile station phone number for example in a database containing user data, like PHR database, and sent 104a the response also or only to the user's mobile station 110a.

The functions of the server, such as interpreting of received ID information, content information and calculations may be performed for example by a server application (implemented e.g. by a suitable computer program) at the server.

The identifier ID as well as user ID can be transferred from the user related communication devices to the server by the application in many different ways like an SMS, voice or other similar message or data connection. Also the server may send the response in similar ways to the user related communication devices.

The invention has been explained above with reference to the aforementioned embodiments, and several advantages of the invention have been demonstrated. It is clear that the invention is not only restricted to these embodiments, but comprises all possible embodiments within the scope of the inventive thought and the following patent claims.

Numbered clauses 1-20:
1. An arrangement for monitoring a personal intake of products, wherein
   - the arrangement comprises identifiers each associated to a product, said identifier comprising readable identifier ID related to said identifier,
   - the arrangement comprises a user related communication device having reading means for reading said identifier ID and sending means for transferring said read identifier ID to a server as a query, said server being in a data communication connect with a first database, where said first database comprises identifier ID and content information of each product or product type,
   - the arrangement is configured to connect said transferred identifier ID with content information of the product or product type with corresponding identifier ID at the first database and to send said content information as a response to said query to said user related communication device.
2. An arrangement according to numbered clause 1, wherein the user related communication device is configured in addition to read identifier ID also to transfer user ID to the server, whereupon the arrangement is configured to keep a consumption database of the consumption of said user by associating said user ID with content information of the product corresponding to said transferred identifier ID, where the server is in data communication connection with said consumption database, and the arrangement is configured to transfer user related consumption information derived from the consumption database, e.g. personal cumulative value or personal daily value, based on said transferred user ID as a respond to the user related communication device and/or to a second database to which the user has access.
3. An arrangement according to numbered clause 2, wherein the consumption database further comprises additional information of the user, such as
   - original weight, age, height, sex, current activity level and how much weight is desired to lose, accumulated radiation dose and/or toxic substances,
   - and based on this additional information as well as content information of the product related to said transferred identifier ID, is configured to calculate against said user ID
      ○ an indication of the calories intake, such as calories still left for that day and/or
      ○ an indication of radiation dose, and/or amounts of toxic
   - and is configured to transfer said indication to the user related communication device and/or to the second database.
4. An arrangement according to numbered clause 1, wherein the user related communication device is a mobile station and/or cash machine.
5. An arrangement according to numbered clause 1, wherein the arrangement is configured to gather content information relating to plurality of products corresponding plurality of identifier IDs and provide content information relating to said plurality of products as cumulative data.
6. An arrangement according to numbered clause 2, wherein the user ID is ID information of the user related communication device, ID information input to the user related communication device or ID information read from the outer source, such as an ID card, and wherein said ID information is e.g. phone number, user identification number, IP-number of the user related communication device, social security number and/or user name.
7. An arrangement according to numbered clause 1, wherein the identifier associated to products is an RFID tag, bar code or smart code (*2D bar code*), and wherein the user related communication device comprises RFID reader, bar code reader, and/or smart code reader.
8. An intake monitoring method for monitoring a personal intake of products, the method comprising
   - associating an identifier to a product, said identifier comprising readable ID information related to said identifier,
   - reading and transferring said identifier ID to a server as a query by a user related communication device, said server being in a communication connect with a first database, where said first database comprises identifier ID and content information of each product or product type,
   - connecting said transferred identifier ID with content information of the product or product type with corresponding identifier ID at the first database and transferring said content information as a response to said query to said user related communication device.
9. A method according to numbered clause 8, comprising:
   - transferring in addition to read identifier ID also user ID to the server,
   - keeping a consumption database of the consumption of said user by associating said user ID with content information of the product corresponding to said transferred identifier ID, the server being in data communication connect with said database and
   - transferring user related consumption information derived from the consumption database, e.g. personal cumulative value or personal daily value, based on said transferred user ID as a respond to the user related communication device and/or to a second database to which the user has access.
10. A method according to numbered clause 9, wherein the consumption database further comprises additional information of the user, such as
   - original weight, age, height, sex, current activity level and how much weight is desired to lose, accumulated radiation dose and/or toxic substances,
   the method comprising
   - calculating, based on these additional information as well as content information of the product related to said transferred identifier ID,
      ○ an indication of the calories intake, such as calories still left for that day and/or
      ○ an indication of radiation dose, and/or amounts of toxic, and
   - transferring said indication to the user related communication device and/or to the second database.
11. A method according to numbered clause 8, wherein content information relating to plurality of products corresponding plurality of identifier IDs is gathered and content information relating to said plurality of products is provided as cumulative data.
12. A user related communication device for monitoring a personal intake of products, wherein the user related communication device is configured to
   - read identifier ID, said identifier being associated to a product and comprising readable identifier ID related to said identifier,
   - send said read identifier ID to a server as a query, said server being in a data communication connect with a first database comprising identifier ID and content information of each product or product type,
   - receive content information as a response to said query, where said content information is provided by connecting said transferred identifier ID with content information of the product or product type with corresponding identifier ID at the first database.
13. A device according to numbered clause 12, wherein the user related communication device is configured to transfer in addition to read identifier ID also user ID to the server, where the server is configured to keep a consumption database of the consumption of said user by associating said user ID with content information of the product corresponding to said transferred identifier ID, and the user related communication device is configured to receive and display user related consumption information, e.g. personal cumulative value or personal daily value, sent by the server and derived from the consumption database based on said transferred user ID.
14. A device according to numbered clause 12, wherein the user related communication device is a mobile station and/or cash machine.
15. A device according to numbered clause 13, wherein the user ID is ID information of the user related communication device, ID information input to the user related communication device or ID information read from the outer source, such as an ID card, and wherein said ID information is e.g. phone number, user identification number, IP-number of the user related communication device, social security number and/or user name.
16. A server for monitoring a personal intake of products, wherein the server is configured to
   - receive an identifier ID as a query sent by a user related communication device, where said ID relates to an identifier associated to one product,
   - be in a data communication connect with a first database, where said first database comprises identifier ID and content information of each product or product type,
   - connect said transferred identifier ID with content information of the product or product type with corresponding identifier ID at the first database and send said content information as a response to said query to said user related communication device.
17. A server according to numbered clause 16, wherein the server is configured to
   - receive in addition to read identifier ID also user ID,
   - keep a consumption database of the consumption of said user by associating said user ID with content information of the product corresponding to said received identifier ID,
   - being in data communication connection with said database, and
   - transfer user related consumption information derived from the consumption database, e.g. personal cumulative value or personal daily value, based on said transferred user ID as a respond to the user related communication device and/or to a second database to which the user has access.
18. A server according to numbered clause 17, wherein the consumption database further comprises additional information of the user, such as
   - original weight, age, height, sex, current activity level and how much weight is desired to lose, accumulated radiation dose and/or toxic substances,
   - and based on these additional information as well as content information of the product related to said transferred identifier ID, the server is configured to calculate against said user ID
      ○ an indication of the calories intake, such as calories still left for that day and/or
      ○ an indication of radiation dose, and/or amounts of toxic
   - and the server is configured to transfer said indication to the user related communication device and/or to the second database.
19. A computer readable medium for monitoring a personal intake of products, wherein the computer readable medium is configured to
   - interpret identifier ID read by a user related communication device, said identifier being associated to a product and comprising readable identifier ID related to said identifier,
   - send said read identifier ID to a server as a query, said server being in a data communication connect with a first database comprising identifier ID and content information of each product or product type,
   - receive content information as a response to said query, where said content information is provided by connecting said transferred identifier ID with content information of the product or product type with corresponding identifier ID at the first database,
   when said computer readable medium is run on the user related communication device.
20. A computer readable medium for monitoring a personal intake of products, wherein the computer readable medium is configured to
   - receive an identifier ID as a query sent by a user related communication device, where said ID relates to an identifier associated to one product,
   - communicating with a first database, where said first database comprises identifier ID and content information of each product or product type, and
   - connecting said transferred identifier ID with content information of the product or product type with corresponding identifier ID at the first database and send said content information as a response to said query to said user related communication device,
when said computer readable medium is run on the server.

## Claims

1. An arrangement for monitoring a personal intake of products, wherein
- the arrangement comprises identifiers each associated to a product, said identifier comprising readable identifier ID related to said identifier,
- the arrangement comprises a user related communication device having reading means for reading said identifier ID and sending means for transferring said read identifier ID to a server as a query, said server being in a data communication connect with a first database, where said first database comprises identifier ID and content information of each product or product type,
- the arrangement is configured to connect said transferred identifier ID with content information of the product or product type with corresponding identifier ID at the first database and to send said content information as a response to said query to said user related communication device.

2. An arrangement according to claim 1, wherein the user related communication device is configured in addition to read identifier ID also to transfer user ID to the server, whereupon the arrangement is configured to keep a consumption database of the consumption of said user by associating said user ID with content information of the product corresponding to said transferred identifier ID, where the server is in data communication connection with said consumption database, and the arrangement is configured to transfer user related consumption information derived from the consumption database, e.g. personal cumulative value or personal daily value, based on said transferred user ID as a respond to the user related communication device and/or to a second database to which the user has access.

3. An arrangement according to claim 2, wherein the consumption database further comprises additional information of the user, such as
- original weight, age, height, sex, current activity level and how much weight is desired to lose, accumulated radiation dose and/or toxic substances,
- and based on this additional information as well as content information of the product related to said transferred identifier ID, is configured to calculate against said user ID
○ an indication of the calories intake, such as calories still left for that day and/or
○ an indication of radiation dose, and/or amounts of toxic
- and is configured to transfer said indication to the user related communication device and/or to the second database.

4. An arrangement according to any of claims 1-3, wherein the arrangement is configured to gather content information relating to plurality of products corresponding plurality of identifier IDs and provide content information relating to said plurality of products as cumulative data.

5. An intake monitoring method for monitoring a personal intake of products, the method comprising
- associating an identifier to a product, said identifier comprising readable ID information related to said identifier,
- reading and transferring said identifier ID to a server as a query by a user related communication device, said server being in a communication connect with a first database, where said first database comprises identifier ID and content information of each product or product type,
- connecting said transferred identifier ID with content information of the product or product type with corresponding identifier ID at the first database and transferring said content information as a response to said query to said user related communication device.

6. A method according to claim 5, comprising:
- transferring in addition to read identifier ID also user ID to the server,
- keeping a consumption database of the consumption of said user by associating said user ID with content information of the product corresponding to said transferred identifier ID, the server being in data communication connect with said database and
- transferring user related consumption information derived from the consumption database, e.g. personal cumulative value or personal daily value, based on said transferred user ID as a respond to the user related communication device and/or to a second database to which the user has access.

7. A method according to claim 6, wherein the consumption database further comprises additional information of the user, such as
- original weight, age, height, sex, current activity level and how much weight is desired to lose, accumulated radiation dose and/or toxic substances,
the method comprising
- calculating, based on these additional information as well as content information of the product related to said transferred identifier ID,
○ an indication of the calories intake, such as calories still left for that day and/or
○ an indication of radiation dose, and/or amounts of toxic, and
- transferring said indication to the user related communication device and/or to the second database.

8. A method according to any of claims 5-7, wherein content information relating to plurality of products corresponding plurality of identifier IDs is gathered and content information relating to said plurality of products is provided as cumulative data.

9. A user related communication device for monitoring a personal intake of products, wherein the user related communication device is configured to
- read identifier ID, said identifier being associated to a product and comprising readable identifier ID related to said identifier,
- send said read identifier ID to a server as a query, said server being in a data communication connect with a first database comprising identifier ID and content information of each product or product type,
- receive content information as a response to said query, where said content information is provided by connecting said transferred identifier ID with content information of the product or product type with corresponding identifier ID at the first database.

10. A device according to claim 9, wherein the user related communication device is configured to transfer in addition to read identifier ID also user ID to the server, where the server is configured to keep a consumption database of the consumption of said user by associating said user ID with content information of the product corresponding to said transferred identifier ID, and the user related communication device is configured to receive and display user related consumption information, e.g. personal cumulative value or personal daily value, sent by the server and derived from the consumption database based on said transferred user ID.

11. A device according to claim 10, wherein the user ID is ID information of the user related communication device, ID information input to the user related communication device or ID information read from the outer source, such as an ID card, and wherein said ID information is e.g. phone number, user identification number, IP-number of the user related communication device, social security number and/or user name.

12. A server for monitoring a personal intake of products, wherein the server is configured to
- receive an identifier ID as a query sent by a user related communication device, where said ID relates to an identifier associated to one product,
- be in a data communication connect with a first database, where said first database comprises identifier ID and content information of each product or product type,
- connect said transferred identifier ID with content information of the product or product type with corresponding identifier ID at the first database and send said content information as a response to said query to said user related communication device.

13. A server according to claim 12, wherein the server is configured to
- receive in addition to read identifier ID also user ID,
- keep a consumption database of the consumption of said user by associating said user ID with content information of the product corresponding to said received identifier ID,
- being in data communication connection with said database, and
- transfer user related consumption information derived from the consumption database, e.g. personal cumulative value or personal daily value, based on said transferred user ID as a respond to the user related communication device and/or to a second database to which the user has access.

14. A server according to claim 13, wherein the consumption database further comprises additional information of the user, such as
- original weight, age, height, sex, current activity level and how much weight is desired to lose, accumulated radiation dose and/or toxic substances,
- and based on these additional information as well as content information of the product related to said transferred identifier ID, the server is configured to calculate against said user ID
○ an indication of the calories intake, such as calories still left for that day and/or
○ an indication of radiation dose, and/or amounts of toxic
- and the server is configured to transfer said indication to the user related communication device and/or to the second database.

15. A computer program for monitoring a personal intake of products, wherein the computer program comprises program code means adapted to perform any steps of the method of any of claims 5-8, when said computer program is run on the user related communication device or the server.
